# EUROPEAN PATENT APPLICATION

(11) **EP 4 119 587 A1**
(43) Date of publication of application: **18.01.2023**
(21) Application number: 21768658.3
(22) Date of filing: 12.03.2021
(51) Int. Cl.: C08F 218/02, C09D 183/04, C09D 183/07, A61L 33/06

(54) **COATING AGENT AND MEDICAL MATERIAL USING SAME**

(30) Priority: 12.03.2020 JP 2020043014
(71) Applicant: Toray Industries, Inc., Tokyo 103-8666 (JP)
(72) Inventor: USHIRO, Suguru, Otsu-shi, Shiga 520-8558 (JP); IIMORI, Hirokazu, Okazaki-shi, Aichi 444-8522 (JP); SAKAGUCHI, Hirokazu, Otsu-shi, Shiga 520-8558 (JP)
(74) Representative: Kador & Partner PartG mbB
(86) International application number: PCT/JP2021/010029
(87) International publication number: WO 2021/182608

(57) **Abstract**

The present invention aims to provide a coating agent that can be applied to various materials without using radiation, and a medical material using the same. The present invention provides a coating agent containing a copolymer that contains: a monomer unit (unit A) containing a Si-O bond; a vinyl carboxylate monomer unit (unit B); and a monomer unit (unit C) containing a hydrophilic group.

## Description

### TECHNICAL FIELD

The present invention relates to a coating agent and a medical material using the same.

### BACKGROUND ART

Since thrombus adhesion and blood coagulation as well as functional deterioration associated therewith could occur in medical materials (such as separation membranes, tubes and metal members) used in contact with blood and medical devices (such as artificial kidneys, artificial lungs, artificial blood vessels, artificial valves, stents, stent-grafts, catheters, blood circuits, cannulas, blood bags and syringes) including or incorporating the same, it is required to give a high antithrombogenicity to the surfaces of the medical materials. To address such problems, attempts have been made so far to improve antithrombogenicity by hydrophilizing the surfaces of the of medical materials, and various studies have been carried out.

For example, Patent Document 1 discloses a method in which an appropriate amount of polyvinylpyrrolidone, which is a hydrophilic polymer, is incorporated into a polysulfone-based resin porous membrane to give hydrophilicity thereto, thereby reducing the fouling of the membrane.

Patent Documents 2 to 5 each disclose a separation membrane of a polysulfone-based polymer on the surface of which a vinylpyrrolidone/vinyl carboxylate copolymer is immobilized.

Further, Patent Document 6 discloses a vinyl lactam/silyl group-containing monomer copolymer, as a base for a hair styling agent.

### PRIOR ART DOCUMENTS

### [Patent Documents]

[Patent Document 1] JP 61-238834 A
[Patent Document 2] JP 2010-104984 A
[Patent Document 3] JP 2011-173115 A
[Patent Document 4] WO 2016/158388
[Patent Document 5] WO 2018/025772
[Patent Document 6] JP 4-321618 A

### SUMMARY OF THE INVENTION

### PROBLEMS TO BE SOLVED BY THE INVENTION

In the membrane disclosed in Patent Document 1, however, the interaction between the hydrophilic polymer such as polyvinylpyrrolidone and the polysulfone-based resin porous membrane is not strong. Therefore, a large amount of hydrophilic polymer needs to be used, in order to introduce the hydrophilic polymer to the polysulfone-based resin porous membrane in an amount sufficient for the membrane to exhibit antithrombogenicity, which is problematic from the viewpoint of practical use.

In each of Patent Documents 2 to 5, on the other hand, it is expected that the vinyl carboxylate unit constituting the vinylpyrrolidone/vinyl carboxylate copolymer interacts with the separation membrane, which is a hydrophobic substrate, to increase the introduction rate of the copolymer to the surface of the separation membrane, allowing for an efficient hydrophilization.

However, it is necessary to use radiation in order to immobilize the vinylpyrrolidone/vinyl carboxylate copolymer described in each of Patent Documents 2 to 5, on the separation membrane as the substrate. Therefore, materials which can be used for the substrate are limited to those with radiation resistance, and thus there is a room for improvement from the viewpoint of general versatility.

Further, the copolymer described in Patent Document 6 is a water-soluble copolymer for a hair styling agent, and associated with a risk of elution when introduced on the surface of a medical material or the like.

Accordingly, an object of the present invention is to provide a coating agent that can be applied to various materials without using radiation, and a medical material using the same.

### MEANS FOR SOLVING THE PROBLEMS

As a result of intensive studies to solve the problems described above, the present inventors have discovered that antifouling properties, particularly, antithrombogenicity can be given to a substrate, by using a coating agent comprising a copolymer that comprises: a monomer unit containing a Si-O bond; a vinyl carboxylate monomer unit; and a monomer unit containing a hydrophilic group.

That is, the present invention includes the following [1] to [10].
[1] A coating agent comprising a copolymer, the copolymer comprising:
   a monomer unit (unit A) containing a Si-O bond;
   a vinyl carboxylate monomer unit (unit B); and
   a monomer unit (unit C) containing a hydrophilic group.
[2] The coating agent according to [1], wherein the unit A is a monomer unit that contains a group selected from the group consisting of alkylalkoxysilyl groups, tris(trialkylsilyloxy)silyl groups and dialkylsiloxane groups.
[3] The coating agent according to [1] or [2], wherein the unit B is a unit selected from the group consisting of vinyl acetate monomer unit, vinyl propionate monomer unit, vinyl butyrate monomer unit, vinyl pentanoate monomer unit, vinyl pivalate monomer unit and vinyl hexanoate monomer unit.
[4] The coating agent according to any one of [1] to [3], wherein the hydrophilic group is an amide group.
[5] The coating agent according to any one of [1] to [4], wherein the units A, B and C are arranged randomly in the copolymer.
[6] A medical material comprising:
   a substrate; and
   a layer formed on the surface of the substrate by the coating agent according to any one of [1] to [5].
[7] The medical material according to [6], wherein the substrate is made of a material containing an olefinic polymer.
[8] A copolymer comprising:
   a monomer unit (unit A) containing a Si-O bond;
   a vinyl carboxylate monomer unit (unit B); and
   a monomer unit (unit C) containing a hydrophilic group;
   wherein the unit A is a monomer unit that contains a group selected from the group consisting of alkylalkoxysilyl groups, tris(trialkylsilyloxy)silyl groups and dialkylsiloxane groups.
[9] The copolymer according to [8],
   wherein the unit B is a unit selected from the group consisting of vinyl acetate monomer unit, vinyl propionate monomer unit, vinyl butyrate monomer unit, vinyl pentanoatc monomer unit, vinyl pivalate monomer unit and vinyl hexanoate monomer unit; and
   wherein the unit C is vinylpyrrolidone monomer unit, vinylacetamide monomer unit or acrylamide monomer unit.
[10] The copolymer according to [8] or [9], wherein the units A, B and C are arranged randomly in the copolymer.

### EFFECT OF THE INVENTION

According to the coating agent of the present invention, antithrombogenicity can be given to various medical materials.

### MODE FOR CARRYING OUT THE INVENTION

The present invention is described below in detail.

The coating agent of the present invention is characterized by comprising a copolymer, the copolymer comprising: a monomer unit (unit A) containing a Si-O bond; a vinyl carboxylate monomer unit (unit B); and a monomer unit (unit C) containing a hydrophilic group.

The term "coating agent" means a substance to be applied to a material surface in order to give a desired property thereto. The property may be, for example, hydrophilicity, hydrophobicity, water repellency, oil repellency, slidability, durability, antifouling properties, antithrombogenicity, etc., and is mainly antithrombogenicity in the present invention. The antithrombogenicity means a characteristic that inhibits the adhesion of biogenic components contained in blood and body fluids, such as proteins and platelets, that trigger thrombus formation, to a material surface.

The copolymer contained in the coating agent of the present invention is described below. The copolymer contains: a monomer unit (unit A) containing a Si-O bond; a vinyl carboxylate monomer unit (unit B); and a monomer unit (unit C) containing a hydrophilic group.

In the present description, the monomer unit means a repeating unit in a polymer obtained by the polymerization of the corresponding monomer.

The Si-O bond contained in the monomer unit (unit A) (hereinafter, also referred to as "unit A") containing a Si-O bond means a bond between a silicon atom and an oxygen atom. The unit A is expected to have roles to improve the adhesion of the copolymer to the substrate as well as to inhibit the activation of complement components in blood. Based on the above, the number of Si-O bonds contained in the unit A is at least not less than one, and preferably not less than 2. On the other hand, the number of Si-O bonds contained in the unit A is preferably an integer not more than 30, and more preferably an integer not more than 25, from the viewpoint of the handleability of the coating agent.

The unit A may contain a group containing a Si-O bond on the side of the main chain or on the side of a side chain, of the unit A. Considering the ease of copolymerization with the units B and C, however, the group containing a Si-O bond is preferably contained on the side of the side chain of the unit A.

In the unit A, the group containing a Si-O bond may be, for example, an alkylalkoxysilyl group, a tris(trialkylsilyloxy)silyl group, or a dialkylsiloxane group.

The alkylalkoxysilyl group means a group in which at least one or more each of alkyl groups and alkoxy groups are bound to a silicon atom. In a preferred embodiment, the alkylalkoxysilyl group may be a group represented by the following General Formula (I): 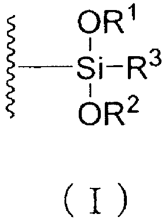 [wherein each of R¹, R² and R³ independently represents an alkyl group having from 1 to 20 carbon atoms or a cycloalkyl group having from 3 to 20 carbon atoms, and the wavy line represents a binding site].

In the General Formula (I) shown above, each of R¹, R² and R³ is preferably independently an alkyl group having from 1 to 10 carbon atoms or a cycloalkyl group having from 3 to 10 carbon atoms, and more preferably independently an alkyl group having from 1 to 4 carbon atoms, so that the coating agent as a whole has a moderate hydrophilicity.

The alkyl group having from 1 to 20 carbon atoms means a linear or branched hydrocarbon group having from 1 to 20 carbon atoms, and examples thereof include methyl group, ethyl group, propyl group, isopropyl group, butyl group, isobutyl group, tert-butyl group, hexyl group, 2-methylhexyl group, decyl group, tetradecyl group, octadecyl group, eicosyl group and cycloeicosyl group.

The cycloalkyl group having from 3 to 20 carbon atoms means a cyclic hydrocarbon group having from 3 to 20 carbon atoms, and examples thereof include cyclopropyl group, methylcyclopropyl group, cyclobutyl group, cyclopentyl group, cyclohexyl group and cycloeicosyl group.

The alkyl group having from 1 to 10 carbon atoms means a linear or branched hydrocarbon group having from 1 to 10 carbon atoms, and examples thereof include methyl group, ethyl group, propyl group, isopropyl group, cyclopropyl group, butyl group, isobutyl group, tert-butyl group, methylcyclopropyl group, cyclobutyl group, hexyl group, 2-methylhexyl group and decyl group.

The cycloalkyl group having from 3 to 10 carbon atoms means a cyclic hydrocarbon group having from 3 to 10 carbon atoms, and examples thereof include cyclopropyl group, methylcyclopropyl group, cyclobutyl group, cyclopentyl group and cyclohexyl group.

The alkyl group having from 1 to 4 carbon atoms means methyl group, ethyl group, propyl group, isopropyl group, cyclopropyl group, butyl group, isobutyl group or tert-butyl group.

Examples of the alkylalkoxysilyl group include methyldimethoxysilyl group (R¹, R² and R³ are methyl groups), ethyldimethoxysilyl group (R¹ and R² are methyl groups, and R³ is an ethyl group) and methyldipropoxysilyl group (R¹ and R² are propyl groups, and R³ is a methyl group). From the viewpoint of availability, the alkylalkoxysilyl group is preferably methyldimethoxysilyl group.

In the unit A, the monomer unit containing the alkylalkoxysilyl group may be, for example, a methacrylic acid alkyl ester monomer unit, an acrylic acid alkyl ester monomer unit, an alkylacrylamide monomer unit or an alkylmethacrylamide monomer unit in which one arbitrary hydrogen atom in the alkyl group in an alkyl ester or an alkyl amide is replaced by the alkylalkoxysilyl group. In the monomer unit described above, some of the hydrogen atoms in the alkyl group may be substituted with a functional group (such as a hydroxyl group), or some of the carbon atom(s) in the alkyl group may be substituted with a hetero atom (such as an oxygen atom). The number of carbon atoms in the alkyl group in the methacrylic acid alkyl ester monomer unit, the acrylic acid alkyl ester monomer unit, the alkylacrylamide monomer unit or the alkylmethacrylamide monomer unit is, for example, from 1 to 6. More specific examples include 3-(methyldimethoxysilyl)ethyl methacrylate monomer unit, 3-(methyldimethoxysilyl)propyl methacrylate monomer unit, 3-(methyldimethoxysilyl)butyl methacrylate monomer unit, 3-(ethyldimethoxysilyl)ethyl methacrylate monomer unit, 3-(ethyldimethoxysilyl)propyl methacrylate monomer unit, 3-(ethyldimethoxysilyl)butyl methacrylate monomer unit, 3-(methyldipropoxysilyl)ethyl acrylate monomer unit, 3-(methyldipropoxysilyl)propyl acrylate monomer unit and 3-(methyldipropoxysilyl)butyl acrylate monomer unit. From the viewpoint of availability, 3-(methyldimethoxysilyl)propyl methacrylate monomer unit or 3-(methyldimethoxysilyl)propyl acrylate monomer unit is preferred.

The tris(trialkylsilyloxy)silyl group means a group in which three trialkylsilyloxy groups are bound to a silicon atom. The three trialkylsilyloxy groups may be the same as, or different from, each other, and the three alkyl groups may be the same as, or different from, each other. In a preferred embodiment, the tris(trialkylsilyloxy)silyl group may be a group represented by the following General Formula (II): 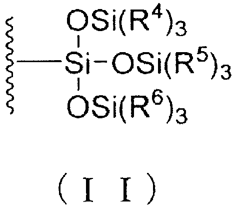 [wherein each of R⁴, R⁵ and R⁶ independently represents an alkyl group having from 1 to 20 carbon atoms or a cycloalkyl group having from 3 to 20 carbon atoms, and the wavy line represents a binding site].

In the General Formula (II) shown above, each of R⁴, R⁵ and R⁶ is preferably independently an alkyl group having from 1 to 10 carbon atoms or a cycloalkyl group having from 3 to 10 carbon atoms, and more preferably independently an alkyl group having from 1 to 4 carbon atoms, so that the coating agent as a whole has a moderate hydrophilicity.

Examples of the tris(trialkylsilyloxy)silyl group include tris(trimethylsilyloxy)silyl group (R⁴s, R⁵s and R⁶s are methyl groups), and di(trimethylsilyloxy)(triethylsilyloxy)silyl group (R⁴s and R⁵s are methyl groups, and R⁶s are ethyl groups). From the viewpoint of availability, all of R⁴s, R⁵s and R⁶s are preferably the same alkyl group, and the tris(trialkylsilyloxy)silyl group is particularly preferably tris(trimethylsilyloxy)silyl group.

In the unit A, the monomer unit containing a tris(trialkylsilyloxy)silyl group may be, for example, a methacrylic acid alkyl ester monomer unit, an acrylic acid alkyl ester monomer unit, an alkylacrylamide monomer unit or an alkylmethacrylamide monomer unit in which one arbitrary hydrogen atom in the alkyl group in an alkyl ester or an alkyl amide is replaced by the tris(trialkylsilyloxy)silyl group. In the monomer unit described above, some of the hydrogen atoms in the alkyl group may be substituted with a functional group (such as a hydroxyl group), or some of the carbon atom(s) in the alkyl group may be substituted with a hetero atom (such as an oxygen atom). The number of carbon atoms in the alkyl group in the methacrylic acid alkyl ester monomer unit, the acrylic acid alkyl ester monomer unit, the alkylacrylamide monomer unit or the alkylmethacrylamide monomer unit is, for example, from 1 to 6. More specific examples include 3-[tris(trimethylsilyloxy)silyl]ethyl methacrylate monomer unit, 3-[tris(trimethylsilyloxy)silyl]propyl methacrylate monomer unit, 3-[tris(trimethylsilyloxy)silyl]butyl methacrylate monomer unit, 3-[di(trimethylsilyloxy)(triethylsilyloxy)silyl]ethyl acrylate monomer unit, 3-[di(trimethylsilyloxy)(triethylsilyloxy)silyl]propyl acrylate monomer unit, 3-[di(trimethylsilyloxy)(triethylsilyloxy)silyl]butyl acrylate monomer unit, 3-[tris(trimethylsilyloxy)silyl]ethyl acrylate monomer unit, 3-[tris(trimethylsilyloxy)silyl]propyl acrylate monomer unit, 3-[tris(trimethylsilyloxy)silyl]butyl acrylate monomer unit, acrylamide 3-[tris(trimethylsilyloxy)silyl]ethyl monomer unit, acrylamide 3-[tris(trimethylsilyloxy)silyl]propyl monomer unit and acrylamide 3-[tris(trimethylsilyloxy)silyl]butyl monomer unit. From the viewpoint of availability, 3-[tris(trimethylsilyloxy)silyl]propyl methacrylate monomer unit or 3-[tris(trimethylsilyloxy)silyl]propyl acrylate monomer unit is preferred.

The dialkylsiloxane group means a group composed of a repeating unit containing a siloxane bond in which two alkyl groups are bound to a silicon atom. The two alkyl groups may be the same as, or different from, each other. In a preferred embodiment, the dialkylsiloxane group may be a group represented by the following General Formula (III): 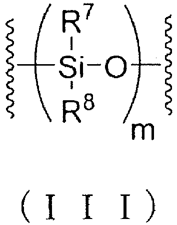 [wherein each of R⁷ and R⁸ independently represents an alkyl group having from 1 to 10 carbon atoms or a cycloalkyl group having from 3 to 10 carbon atoms, m represents an integer not less than 1, and each wavy line represents a binding site].

In General Formula (III), each of R⁷ and R⁸ is preferably independently an alkyl group having from 1 to 4 carbon atoms, so that the coating agent as a whole has a moderate hydrophilicity.

m is preferably an integer from 1 to 100, more preferably an integer from 1 to 20, and still more preferably an integer from 1 to 10, so that the coating agent as a whole has a moderate hydrophilicity.

Examples of the dialkylsiloxane group include dimethylsiloxane group (R⁷ and R⁸ are methyl groups), diethylsiloxane group (R⁷ and R⁸ are ethyl groups) and isopropylbutylsiloxane group (R⁷ is an isopropyl group, and R⁸ is a butyl group). From the viewpoint of availability, R⁷ and R⁸ are preferably the same alkyl group, and the dialkylsiloxane group is particularly preferably dimethylsiloxane group or diethylsiloxane group.

The dialkylsiloxane group may be a group represented by the following General Formula (IV): 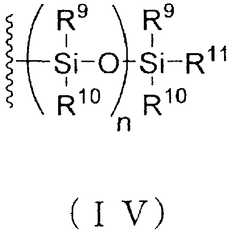 [wherein each of R⁹ and R¹⁰ independently represents an alkyl group having from 1 to 10 carbon atoms or a cycloalkyl group having from 3 to 10 carbon atoms, R¹¹ represents an alkyl group having from 1 to 10 carbon atoms, n represents an integer from 1 to 10, and the wavy line represents a binding site].

In General Formula (IV), each of R⁹ and R¹⁰ is preferably independently an alkyl group having from 1 to 4 carbon atoms.

In General Formula (IV), R¹¹ is preferably an alkyl group having from 1 to 4 carbon atoms.

n is preferably an integer from 1 to 4, from the viewpoint of controlling the hydrophobicity of the coating agent as a whole.

Examples of the dialkylsiloxane group include
5-methyl-1,1,3,3,5,5-hexaethyl-1-trisiloxanyl group (R⁹s and R¹⁰s are ethyl groups, R¹¹ is a methyl group, and n = 2), and
9-butyl-1,1,3,3,5,5,7,7,9,9-decamethyl-1-pentasiloxanyl group (R⁹s and R¹⁰s are methyl groups, R¹¹ is a butyl group, and n = 4). From the viewpoint of availability, R⁹s and R¹⁰s are preferably methyl groups.

In the unit A, the monomer unit containing a dialkylsiloxane group may be, for example, a methacrylic acid alkyl ester monomer unit, an acrylic acid alkyl ester monomer unit, an alkylacrylamide monomer unit or an alkylmethacrylamide monomer unit in which one arbitrary hydrogen atom in the alkyl group in an alkyl ester or an alkyl amide is replaced by the dialkylsiloxane group. In the monomer unit described above, some of the hydrogen atoms in the alkyl group may be substituted with a functional group (such as a hydroxyl group), or some of the carbon atom(s) in the alkyl group may be substituted with a hetero atom (such as an oxygen atom). The number of carbon atoms in the alkyl group in the methacrylic acid alkyl ester monomer unit, the acrylic acid alkyl ester monomer unit, the alkylacrylamide monomer unit or the alkylmethacrylamide monomer unit is, for example, from 1 to 6. More specific examples include
3-[3-(5-methyl-1,1,3,3,5,5-hexaethyl-1-trisiloxanyl)propoxyl]-2-hydroxylethyl acrylate monomer unit,
3-[3-(5-methyl-1,1,3,3,5,5-hexaethyl-1-trisiloxanyl)propoxyl]-2-hydroxylpropyl acrylate monomer unit,
3-[3-(5-methyl-1,1,3,3,5,5-hexaethyl-1-trisiloxanyl)propoxyl]-2-hydroxylbutyl acrylate monomer unit,
3-[3-(5-methyl-1,1,3,3,5,5-hexaethyl-1-trisiloxanyl)propoxyl]-2-hydroxylethyl methacrylate monomer unit,
3-[3-(5-methyl-1,1,3,3,5,5-hexaethyl-1-trisiloxanyl)propoxyl]-2-hydroxylpropyl methacrylate monomer unit,
3-[3-(5-methyl-1,1,3,3,5,5-hexaethyl-1-trisiloxanyl)propoxyl]-2-hydroxylbutyl methacrylate monomer unit,
3-[3-(9-butyl-1,1,3,3,5,5,7,7,9,9-decamethyl-1-pentasiloxanyl)propoxyl]-2-hydroxyle thyl acrylate monomer unit,
3-[3-(9-butyl-1,1,3,3,5,5,7,7,9,9-decamethyl-1-pentasiloxanyl)propoxyl]-2-hydroxyl propyl acrylate monomer unit,
3-[3-(9-butyl-1,1,3,3,5,5,7,7,9,9-decamethyl-1-pentasiloxanyl)propoxyl]-2-hydroxyl butyl acrylate monomer unit,
3-[3-(9-butyl-1,1,3,3,5,5,7,7,9,9-decamethyl-1-pentasiloxanyl)propoxyl]-2-hydroxyle thyl methacrylate monomer unit,
3-[3-(9-butyl-1,1,3,3,5,5,7,7,9,9-decamethyl-1-pentasiloxanyl)propoxyl]-2-hydroxyl propyl methacrylate monomer unit, and
3-[3-(9-butyl-1,1,3,3,5,5,7,7,9,9-decamethyl-1-pentasiloxanyl)propoxyl]]-2-hydroxyl butyl methacrylate monomer unit. From the viewpoint of availability, 3-[3-(9-butyl-1,1,3,3,5,5,7,7,9,9-decamethyl-1-pentasiloxanyl)propoxyl]-2-hydroxyl propyl methacrylate monomer unit is preferred.

The unit A is not particularly limited as long as it is a monomer unit containing a Si-O bond, and is preferably a monomer unit that contains a group selected from the group consisting of alkylalkoxysilyl groups, tris(trialkylsilyloxy)silyl groups and dialkylsiloxane groups. The unit A is more preferably a methacrylic acid alkyl ester monomer unit, an acrylic acid alkyl ester monomer unit, an alkylacrylamide monomer unit or an alkylmethacrylamide monomer unit in which one arbitrary hydrogen atom in the alkyl group in an alkyl ester or an alkyl amide is replaced by the group selected from the group consisting of alkylalkoxysilyl groups, tris(trialkylsilyloxy)silyl groups and dialkylsiloxane groups. The unit A is still more preferably 3-(methyldimethoxysilyl)propyl methacrylate monomer unit, 3-(methyldimethoxysilyl)propyl acrylate monomer unit, 3-[tris(trimethylsilyloxy)silyl]propyl methacrylate monomer unit, 3-[tris(trimethylsilyloxy)silyl]propyl acrylate monomer unit, 3-[tris(trimethylsilyloxy)silyl]propyl methacrylate monomer unit or 3-[3-(9-buty]-1,1,3,3,5.5,7,7,9,9-decamethyl-1-pentasiloxanyl)propoxyl]-2-hydroxyl propyl methacrylate monomer unit.

An embodiment of the unit A other than those described above may be, for example, a monomer unit containing a polyhedral oligomeric silsesquioxane (PSS) group. Specific examples thereof include propylmethacryl-heptamethyl-PSS monomer unit, propylmethacryl-heptaisobutyl-PSS monomer unit and propylmethacryl-heptaisobutyl-PSS monomer unit.

The vinyl carboxylate monomer unit (unit B) (hereinafter, also referred to as "unit B") is a monomer unit containing a structure (-CH(OCO-R)-CH₂-) (wherein R represents a hydrocarbon group in which an arbitrary hydrogen atom may be substituted by another atom) derived from a carboxylic acid vinyl ester, wherein R is preferably a hydrocarbon group having from 1 to 20 carbon atoms, and more preferably a hydrocarbon group having from 1 to 8 carbon atoms, and still more preferably a hydrocarbon group having from 2 to 5 carbon atoms. The hydrocarbon group herein is preferably an alkyl group.

The unit B is preferably a unit selected from the group consisting of vinyl acetate monomer unit, vinyl propionate monomer unit, vinyl butyrate monomer unit, vinyl pentanoate monomer unit, vinyl pivalate monomer unit and vinyl hexanoate monomer unit. This is thought to be because the introduction of a flexible vinyl carboxylate monomer unit provides a moderate mobility to the resulting copolymer, and inhibits the adhesion of biogenic components thereto. The unit B is more preferably vinyl propionate monomer unit, vinyl butyrate monomer unit or vinyl pivalate unit, due to having a hydrophobicity that is not too high.

The monomer unit (unit C) (hereinafter, also referred to as "unit C") containing a hydrophilic group is not particularly limited as long as it is a monomer unit containing any of the following hydrophilic groups.

The hydrophilic group to be contained in the unit C means a group capable of forming a hydrogen bond with a water molecule or between functional groups. The hydrophilic group is preferably a group selected from the group consisting of amide group, hydroxyl group, carboxy group, alkylene glycol group, amino group, sulfonic acid group and betaine group. The hydrophilic group may be a free base, a salt or in an ionized form. A part of the hydrophilic group may be used in a bond with another functional group. Since proteins and platelets in blood easily adhere to a highly hydrophobic substrate surface, the unit C has roles to reduce the hydrophobicity and to improve the hydrophilicity, of the substrate surface. The unit C is required to contain at least one kind of hydrophilic group, and may contain a plurality of the same hydrophilic groups or a plurality of different kinds of hydrophilic groups. In particular, the hydrophilic group to be contained in the unit C is preferably a group selected from the group consisting of amide group, hydroxyl group, carboxy group and alkylene glycol group, and more preferably an amide group, from the viewpoints of having a hydrophilicity that is not extremely too high, and being easily available. That is, the unit C is preferably a monomer unit containing a group selected from the group consisting of amide group, hydroxyl group, carboxy group and alkylene glycol group as a hydrophilic group, and more preferably a monomer unit containing an amide group as a hydrophilic group.

In the unit C, the monomer unit containing an amide group as a hydrophilic group may be, for example, a vinyl lactam monomer unit, vinylacetamide monomer unit, a vinylacetamide derivative monomer unit, acrylamide monomer unit, an acrylamide derivative monomer unit, methacrylamide monomer unit or a methacrylamide derivative monomer unit. The derivative herein means that an arbitrary hydrogen atom is replaced by a hydrocarbon group (such as an alkyl group). An arbitrary hydrogen atom in the hydrocarbon group may be replaced by another atom. The number of carbon atoms in the hydrocarbon group is preferably an integer from 1 to 5, and more preferably an integer from 1 to 3.

The vinyl lactam monomer unit means a monomer unit having a cyclic amide structure, and examples thereof include vinylpyrrolidone monomer unit and vinyl caprolactam monomer unit.

The vinylacetamide monomer unit means a monomer unit having the vinylacetamide structure (-CH₂-CH(NH-CO-CH₃)-).

The vinylacetamide derivative monomer unit means a monomer unit having a vinylacetamide-derived structure (-CH₂-CH(NR^{a}-CO-CH₃)-) (wherein R^{a} is a hydrocarbon group, and an arbitrary hydrogen atom in the hydrocarbon group may be replaced by another atom), and examples thereof include N-alkyl-N-vinylacetamide monomer units such as N-methyl-N-vinylacetamide monomer unit and N-ethyl-N-vinylacetamide monomer unit.

The acrylamide monomer unit means a monomer unit having the acrylamide-derived structure (-CH₂-CH(CO-NH₂)-).

The acrylamide derivative monomer unit means a monomer unit having an acrylamide-derived structure (-CH₂-CH(CO-NR^{b}R^{c})-) (wherein each of R^{b} and R^{c} independently represents a hydrogen atom or a hydrocarbon group, and an arbitrary hydrogen atom in the hydrocarbon group may be replaced by another atom, with the proviso that R^{b} and R^{c} are not simultaneously hydrogen atoms), and examples thereof include N-alkylacrylamide monomer units such as N-methylacrylamide monomer unit, N-isopropylacrylamide monomer unit and N-tert-butylacrylamide monomer unit; and N,N-dialkylacrylamide monomer units such as N,N-dimethylacrylamide monomer unit and N,N-diethylacrylamide monomer unit.

The methacrylamide monomer unit means a monomer unit having the methacrylamide-derived structure (-CH₂-C(CH₃)(CO-NH₂)-).

The methacrylamide derivative monomer unit means a monomer unit having a methacrylamide-derived structure (-CH₂-C(CH₃)(CO-NR^{d}R^{e})-) (wherein each of R^{d} and R^{e} independently represents a hydrocarbon group, and an arbitrary hydrogen atom in the hydrocarbon group may be replaced by another atom, with the proviso that R^{d} and R^{e} are not simultaneously hydrogen atoms), and examples thereof include N-alkylmethacrylamide monomer units such as N-isopropylmethacrylamide monomer unit.

In the unit C, examples of the monomer unit containing a hydroxyl group as a hydrophilic group include vinyl alcohol monomer unit and 2-hydroxyethyl methacrylate monomer unit.

In the unit C, examples of the monomer unit containing a carboxy group as a hydrophilic group include acrylic acid monomer unit and methacrylic acid monomer unit.

In the unit C, examples of the monomer unit containing an alkylene glycol group as a hydrophilic group include 2-methylethoxyethyl acrylate monomer unit and 2-(2-ethoxyethoxy)ethyl acrylate monomer unit.

In the unit C, examples of the monomer unit containing an amino group as a hydrophilic group include allylamine hydrochloride monomer unit and 4-amino styrene monomer unit.

In the unit C, examples of the monomer unit containing a sulfonic acid as a hydrophilic group include potassium 3-sulfopropyl acrylate monomer unit.

In the unit C, examples of the monomer unit containing a betaine group as a hydrophilic group include 4-[(3-methacrylamidopropyl)dimethylammonio]butane-1-sulfonate monomer unit.

The unit C is preferably a vinyl lactam monomer unit, vinylacetamide monomer unit, a vinylacetamide derivative monomer unit, acrylamide monomer unit or an acrylamide derivative monomer unit, because of the ease of copolymerization with the monomers of the unit A and the unit B. The unit C is more preferably vinylpyrrolidone monomer unit, vinylacetamide monomer unit or acrylamide monomer unit, because of its excellent biocompatibility.

The above-described copolymer contains at least one each of the unit A, the unit B and the unit C, but may contain a monomer unit other than the units A, B and C, as long as the effect of the invention is not impaired. Examples of the other monomer unit include: monomer units of general-purpose polymers, such as ethylene monomer unit and styrene monomer unit; monomer units containing a halogen atom, such as 1H,1H,2H,2H-heptadecafluorodecyl acrylate monomer unit and vinylidene chloride monomer unit; and monomer units of biodegradable polymers, such as lactic acid monomer unit and glycolic acid monomer unit.

In the copolymer, preferred embodiments of the unit A, the unit B and the unit C can be arbitrarily combined. Examples of such a combination include: a combination of a monomer unit that contains a group selected from the group consisting of alkylalkoxysilyl groups, tris(trialkylsilyloxy)silyl groups and dialkylsiloxane groups, a vinyl carboxylate monomer unit, and a monomer unit containing an amide group; and a combination of a monomer unit that contains a group selected from the group consisting of alkylalkoxysilyl groups, tris(trialkylsilyloxy)silyl groups and dialkylsiloxane groups, a vinyl carboxylate monomer unit which is a unit selected from the group consisting of vinyl acetate monomer unit, vinyl propionate monomer unit, vinyl butyrate monomer unit, vinyl pentanoate monomer unit, vinyl pivalate monomer unit and vinyl hexanoate monomer unit, and vinylpyrrolidone monomer unit, vinylacetamide monomer unit or acrylamide monomer unit. Specific examples thereof include: a combination of a monomer unit containing an alkylalkoxysilyl group, vinyl propionate monomer unit and vinylpyrrolidone monomer unit; a combination of a monomer unit containing a tris(trialkylsilyloxy)silyl group, vinyl propionate monomer unit and vinylpyrrolidone monomer unit; a combination of a monomer unit containing a tris(trialkylsilyloxy)silyl group, vinyl hexanoate monomer unit and vinylpyrrolidone monomer unit; a combination of a monomer unit containing a tris(trialkylsilyloxy)silyl group, vinyl hexanoate monomer unit and vinylacetamide monomer unit; a combination of a monomer unit containing a dialkylsiloxane group, vinyl propionate monomer unit and vinylpyrrolidone monomer unit; a combination of a monomer unit containing a dialkylsiloxane group, vinyl acetate monomer unit and vinylpyrrolidone monomer unit; a combination of a monomer unit containing an alkylalkoxysilyl group, vinyl butyrate monomer unit and vinylpyrrolidone monomer unit; and a combination of a monomer unit containing a tris(trialkylsilyloxy)silyl group, vinyl pivalate monomer unit and acrylamide monomer unit. More specific examples thereof include: a combination of 3-(methyldimethoxysilyl)propyl methacrylate monomer unit, vinyl propionate monomer unit and vinylpyrrolidone monomer unit; a combination of 3-[tris(trimethylsilyloxy)silyl]propyl methacrylate monomer unit, vinyl propionate monomer unit and vinylpyrrolidone monomer unit; a combination of 3-[tris(trimethylsilyloxy)silyl]propyl methacrylate monomer unit, vinyl hexanoate monomer unit and vinylpyrrolidone monomer unit; a combination of 3-[tris(trimethylsilyloxy)silyl]propyl methacrylate monomer unit, vinyl hexanoate monomer unit and vinylacetamide monomer unit; a combination of 3-[3-(9-butyl-1,1,3,3,5,5,7,7,9,9-decamethyl-1-pentasiloxanyl)propoxyl]-2-hydroxyl propyl methacrylate monomer unit, vinyl propionate monomer unit and vinylpyrrolidone monomer unit; a combination of dimethylsiloxane monomer unit, vinyl propionate monomer unit and vinylpyrrolidone monomer unit; a combination of 3-[3-(9-butyl-1,1,3,3,5,5,7,7,9,9-decamethyl-1-pentasiloxanyl)propoxyl]-2-hydroxyl propyl methacrylate monomer unit, vinyl acetate monomer unit and vinylpyrrolidone monomer unit; a combination of 3-(methyldimethoxysilyl)propyl methacrylate monomer unit, vinyl butyrate monomer unit and vinylpyrrolidone monomer unit; and a combination of 3-[tris(trimethylsilyloxy)silyl]propyl methacrylate monomer unit, vinyl pivalate monomer unit and acrylamide monomer unit.

The units A, B and C in the copolymer are preferably arranged randomly or blockwise.

The expression "arranged randomly" means that the units A, B and C are bound at random, in the primary structure of the copolymer.

The expression "arranged blockwise" means that at least one of the units A, B and C arc bound consecutively in not less than 10 units, in the primary structure of the copolymer. One example of the blockwise arrangement is a graft copolymer composed of a main-chain block composed of the unit A, and a branch block(s) composed of the units B and C. The combination of the units constituting the main-chain block and the branch block(s) is not particularly limited. From the viewpoint of the ease of synthesis, however, it is preferred that the main-chain block be composed of the unit A, the branch block(s) be composed of the units B and C, and the units B and C be arranged randomly in the branch block(s).

The units A, B and C are preferably arranged randomly in the copolymer, from the viewpoint of preventing the respective components to aggregate with each other to induce adhesion of proteins and platelets.

The copolymer can be synthesized by a known method. For example, the copolymer can be synthesized by the chain polymerization method represented by the radical polymerization method using a radical polymerization initiator, using the respective source monomers of the unit A, the unit B and the unit C.

Each source monomer of the unit A may be a commercially available product, or may be synthesized by a known method.

In cases where the unit A is the monomer unit containing an alkylalkoxysilyl group, the source monomer thereof can be synthesized, for example, by a hydrosilylation reaction between a hydrosilane compound containing an Si-H group and an unsaturated compound containing a polymerizable group such as a (meth)acrylic group.

In cases where the unit A is the monomer unit containing a tris(trialkylsilyloxy)silyl group, the source monomer thereof can be synthesized, for example, by subjecting acrylic acid or methacrylic acid to an alkylation reaction by an alkyl halide compound containing a tris(trialkylsilyloxy)silyl group. The alkyl halide compound containing a tris(trialkylsilyloxy)silyl group may be a commercially available product, or may be synthesized by a hydrosilylation reaction between a hydrosilane compound containing a Si-H group and an unsaturated alkyl compound containing a halogen atom.

In cases where the unit A is the monomer unit containing a dialkylsiloxane group, the source monomer thereof can be synthesized, for example, by subjecting acrylic acid or methacrylic acid to an alkylation reaction by a hydroxylated alkyl compound containing a dialkylsiloxane group.

As the respective source monomers of the unit B and the unit C, commercially available products can be used.

The copolymer in which the units A, B and C are arranged randomly can be produced, for example, by the following production method, but not limited thereto.

The respective monomers, a polymerization solvent and a polymerization initiator are mixed, and heated to a predetermined temperature under a nitrogen atmosphere. Thereafter, the mixture is mixed with stirring for a predetermined period of time to perform a polymerization reaction. The reaction liquid is cooled to room temperature to terminate the polymerization reaction, and then introduced into a solvent such as water. The copolymer can be obtained by collecting the precipitates formed, followed by drying.

The polymerization reaction is carried out preferably at a reaction temperature of from 30 to 100°C, more preferably from 45 to 90°C, and still more preferably from 60 to 80°C. Further, the polymerization reaction is carried out preferably under normal pressure.

The reaction time of the polymerization reaction is selected as appropriate depending on the conditions such as reaction temperature, and is preferably not less than 1 hour, more preferably not less than 2 hours, and still more preferably not less than 3 hours. It is possible to prevent the monomers from remaining after the polymerization reaction, by avoiding too short reaction time. On the other hand, the reaction time is preferably not more than 10 hours, and more preferably not more than 8 hours. It is possible to prevent side reactions such as the generation of dimers and to more easily control the molecular weight, by avoiding too long reaction time.

The polymerization solvent to be used in the polymerization reaction is not particularly limited as long as the solvent is compatible with the monomers. Examples of the polymerization solvent include: ether solvents such as dioxane and tetrahydrofuran; amide solvents such as N,N-dimethylformamide; sulfoxide solvents such as dimethyl sulfoxide; aromatic hydrocarbon solvents such as benzene and toluene; and alcohol solvents such as methanol, ethanol, isopropyl alcohol, amyl alcohol and hexanol. An ether solvent or an alcohol solvent is preferably used, because the monomers are well soluble in such a solvent.

The polymerization initiator to be used in the polymerization reaction may be, for example, a polymerization initiator that generates radicals, cations or anions. However, a radical polymerization initiator is suitably used, since side reactions are less likely to occur. Examples of the radical polymerization initiator include: azo initiators such as azobisisobutyronitrile, azobisdimethylvaleronitrile and azobis(isobutyric acid)dimethyl; and peroxide initiators such as hydrogen peroxide, benzoyl peroxide, di-tert-butyl peroxide and dicumyl peroxide.

The solvent into which the polymerization reaction liquid is introduced, after the termination of the polymerization reaction, is not particularly limited as long as the copolymer precipitates in the solvent. Examples of the solvent include: hydrocarbon solvents such as pentane, hexane, heptane, octane, nonane and decane; ether solvents such as dimethyl ether, ethyl methyl ether, diethyl ether and diphenyl ether; and water. Since the coating agent and the copolymer of the present invention are preferably water-insoluble or poorly water-soluble, the solvent is preferably water.

The total of the molar fractions of the unit A, the unit B and the unit C with respect to the total amount of the copolymer is preferably not less than 35% by mole, more preferably not less than 60% by mole, still more preferably not less than 80% by mole, yet still more preferably not less than 90% by mole, and yet still more preferably not less than 95% by mole. The upper limit thereof is 100% by mole.

The molar fraction of the unit A with respect to the total amount of the copolymer is preferably not less than 5% by mole and not more than 70% by mole, more preferably not less than 7% by mole and not more than 65% by mole, and still more preferably not less than 9% by mole and not more than 60% by mole. A higher adhesion of the copolymer to a substrate, and a higher antithrombogenicity can be expected, when the molar fraction of the unit A is within the above-described range. The upper limit value and the lower limit value thereof can be combined freely.

Likewise, the molar fraction of the unit B with respect to the total amount of the copolymer is preferably not less than 10% by mole and not more than 70% by mole, more preferably not less than 12% by mole and not more than 55% by mole, and still more preferably not less than 15% by mole and not more than 45% by mole. The upper limit value and the lower limit value thereof can be combined freely.

The molar fraction of the unit C with respect to the total amount of the copolymer is preferably not less than 20% by mole and not more than 80% by mole, more preferably not less than 25% by mole and not more than 75% by mole, and still more preferably not less than 30% by mole and not more than 70% by mole. The upper limit value and the lower limit value thereof can be combined freely.

Preferred embodiments of the structures of the respective units of the copolymer, preferred embodiments of the arrangements of the respective units thereof, and preferred embodiments of the molar fractions of the respective units thereof can be combined arbitrarily.

The molar fraction described above can be calculated, for example, from the ratio of the peak area of each monomer unit to the peak area of all the monomer units constituting the copolymer, obtained by performing a nuclear magnetic resonance (NMR) measurement. In cases where the molar fraction cannot be calculated by the NMR measurement due to reasons such as the overlapping of the peaks, the molar fraction may be calculated by elemental analysis.

In contrast, the copolymer in which the units A, B and C are arranged blockwise can be synthesized, for example, by separately synthesizing a block composed of the unit A, a block composed of the unit B and a block composed of the unit C in advance, and then allowing the respective blocks to bind with one another.

The coating agent of the present invention and the copolymer contained in the coating agent are preferably water-insoluble or poorly water-soluble, since they are used in contact with blood and the like. Being "water-insoluble" herein means that the solubility of the copolymer in 100 g of water at 20°C is less than 0.1 g. Being "poorly water-soluble" means that that the solubility of the copolymer in 100 g of water at 20°C is less than 5 g, and preferably 1 g.

The coating agent of the present invention contains the copolymer, and may contain, in addition thereto, other additives such as an antibacterial agent, a plasticizer, a crosslinking agent and the like. The mass fraction of the copolymer with respect to the total amount of the coating agent is preferably not less than 50% by mass, more preferably not less than 60% by mass, and still more preferably not less than 70% by mass. The upper limit thereof is 100% by mass.

In cases where the coating agent contains the copolymer alone, the coating agent (the copolymer) may be used as it is, or alternatively, the coating agent (the copolymer) may be dissolved in an appropriate solvent to a predetermined concentration to prepare a coating solution. In cases where the coating agent contains an additive(s) in addition to the copolymer, on the other hand, the coating agent (the copolymer and the additive(s)) may be mixed and used as it is, or alternatively, the copolymer and the additive(s) may be mixed to prepare the coating agent, and then dissolved in an appropriate solvent to prepare a coating solution.

The coating agent of the present invention can be used, for example, but not limited to, for the purposes of glazing, scratch filling, anti-fouling and the like, and is preferably used for the purpose of anti-fouling. In particular, the coating agent is preferably used as a coating agent for a medical material for the purpose of providing antithrombogenicity, since the coating agent inhibits the adhesion of proteins and platelets.

The term "medical material" means a member used in contact with biogenic components. In particular, the medical material is suitably a member used for the storage, separation or detection of a biogenic component, the passage of a body fluid, placement in the body or the like. Examples of such a medical material include films, tubes, separation membranes, bags, casings (containers), wires and needles, which are incorporated in medical devices or constituting a part of medical devices. Here, the medical device is preferably a device used for the treatment (including the treatment, the separation of a biogenic component in the body of an organism and the like) or diagnosis, of an organism (for example, a mammal such as a human).

The term "biogenic component" means a substance derived from an organism, such as a saccharide, a protein, a platelet, a DNA, an RNA, a cell or a virus, and the definition thereof includes a body fluid and an aqueous solution that contain such a substance. Since the medical material is often used in contact with a body fluid of an organism, substances contained in a body fluid such as blood, tear fluid or cerebrospinal fluid are preferred as the target biogenic components. When it is intended to allow exertion of the antithrombogenicity by the coating agent, proteins and platelets contained in blood are preferred as the target substances.

In the medical material of the present invention, the properties (such as antithrombogenicity) of the copolymer contained in the coating agent have been given to a substrate, by forming a layer on the surface of the substrate by the coating agent.

The layer formed by the coating agent is present at least on the surface of the medical material. Further, the layer may be present over the entire substrate, or may be formed so as to be localized on the surface of the substrate. However, the layer is preferably formed so as to be localized on the surface of the substrate, because the medical material can be produced easily. In other words, it is preferred that a coating layer be formed on the surface of the substrate by the coating agent. Further, the layer formed by the coating agent may be present over the entire surface, or may be formed so as to be localized on a part of the surface. From the viewpoint of giving the properties (such as antithrombogenicity) to the entire surface of the medical material, however, the layer is preferably formed at least on the surface that comes into contact with the biogenic components, and more preferably formed over the entire surface. In other words, it is preferred that a coating layer be formed at least on the surface that comes into contact with the biogenic components, by the coating agent, and it is more preferred that a coating layer be formed over the entire surface of the substrate.

The term "substrate" means the portion other than the coating agent, of the components constituting the medical material. That is, the substrate corresponds to the portion of the medical material that is present from before the formation of the layer composed of the coating agent. The substrate is preferably one used for medical supplies, and examples thereof include films, tubes, separation membranes, bags, casings (containers), wires and needles, which are incorporated in medical devices or constituting a part of medical devices. A commercially available product can be used as the substrate.

The fact that the layer formed by the coating agent is present on the substrate surface can be confirmed by time-of-flight secondary ion mass spectrometry (TOF-SIMS) and X-ray photoelectron spectroscopy (XPS). In cases where the composition analysis of the surface of the medical material is carried out by the TOF-SIMS measurement, carboxylic acid ions derived from the unit B are detected. In cases where the XPS measurement is carried out, the peak of the silicon atom derived from the unit A is detected. Further, the peak of the carbon atom derived from the ester group of the unit B is detected, at the C1s peak that indicates the presence of carbon atom. In cases where the unit C contains an amide group as a hydrophilic group, the peak of the carbon atom derived from the amide group of the unit C is detected at the C1s peak. The surface means the portion up to a depth of about 10 nm to be measured by TOF-SIMS and XPS. When the signals derived from the coating agent could be observed by the analysis methods described above, it is determined that the layer is formed on the surface of the substrate.

The medical material of the present invention can be obtained, for example, by bringing the substrate into contact with a solution in which the coating agent is dissolved. That is, in cases where a solution in which the coating agent is dissolved is brought into contact with the substrate of the medical material, to be introduced onto the surface thereof, it is possible to use, for example, a method in which the substrate is immersed in the solution in which the coating agent is dissolved, or a method in which the solution in which the coating agent is dissolved is sprayed onto the substrate. From the viewpoint of simplifying the process, the method in which the substrate is immersed in the solution in which the coating agent is dissolved is preferred.

In the case of using the coating agent, the coating agent can be used as it is, or in a state dissolved in a solvent. After coating the substrate, the substrate can be subjected to washing and drying as necessary, to form a layer on the surface of the substrate.

When the concentration of the coating agent in the solution is too low, it results in a failure to introduce a sufficient amount of the coating agent onto the substrate surface. Accordingly, the concentration of the coating agent in the solution is preferably not less than 1% by mass, more preferably not less than 3% by mass, and still more preferably not less than 5% by mass. Further, the concentration of the coating agent in the solution is preferably not more than 20% by mass, and more preferably not more than 10% by mass.

The solution in which the coating agent is dissolved is brought into contact with the substrate preferably at a temperature of not more than 80°C, and more preferably not more than 50°C or lower, because too high temperature may cause the deterioration of the medical material. When the above-described temperature is high, the hydrophobicity of the copolymer contained in the coating agent is relatively increased, possibly resulting in an increase in the introduction efficiency. Therefore, the temperature of the solution in which the copolymer is dissolved is preferably not less than 10°C, more preferably not less than 15°C, and still more preferably not less than 20°C.

The solution in which the coating agent is dissolved is brought into contact with the substrate preferably for a period of time not less than 10 seconds, more preferably not less than 30 seconds, and still more preferably not less than 1 minute, from the viewpoint of introducing a sufficient amount of the coating agent. Further, the above-described time is preferably not more than 5 hours, more preferably not more than 1 hour, and still more preferably not more than 10 minutes, because there is less risk of deteriorating the substrate.

Since the coating agent of the present invention is preferably insoluble or poorly soluble in water, the coating agent is preferably dissolved in an organic solvent which does not deteriorate the substrate of the medical material, or in a mixed solvent of an organic solvent and water. Examples of the organic solvent include: alcohol solvents such as methanol, ethanol and propanol; and ether solvents such as tetrahydrofuran, but not limited thereto.

The substrate (medical material) which has been brought into contact with the solution in which the coating agent is dissolved is preferably dried, in order to remove the residual solvent. Drying conditions are not particularly limited. However, the substrate is preferably dried under vacuum, from the viewpoint of quickly removing the solvent. Further, the drying is preferably carried out in the temperature range of from 10 to 60°C, since there is less risk of the deterioration of the medical material. The drying time is preferably not less than 1 hour, more preferably not less than 6 hours, and still more preferably not less than 12 hours. The upper limit thereof is not particularly limited. However, the drying time is preferably within 5 days, and more preferably within 2 days, from the viewpoint of reducing the production cost.

Before drying the substrate, a washing step with water or an organic solvent may be carried out for the purpose of removing the coating agent in excess. Further, a crosslinking agent may be incorporated into the coating agent in advance, and a step of heating the coating agent to a predetermined temperature before the drying to allow thermal crosslinking to occur so that the coating agent is fixed more firmly on the substrate, may be carried out.

The material of the substrate in the present invention is preferably a metal or a polymer material from the viewpoint of providing a sufficient strength to the medical material, but not particularly limited thereto.

A highly hydrophobic polymer material is used as the polymer material to be used for the substrate, and examples thereof include olefinic polymers such as polyethylene, polypropylene and polymethylpentene, as well as polycarbonate, polyvinyl chloride and silicone (rubber).

In particular, the coating agent of the present invention can be used for coating a substrate that contains as a material thereof, an olefinic polymer which is highly hydrophobic and which is generally regarded as difficult to be applied to a material surface, and thus is useful.

The olefinic polymer means a polymer composed of carbon atoms and hydrogen atoms, and examples thereof include polyethylene, polypropylene, polymethylpentene, polyisoprene, polybutadiene, polycyclopentadiene and polynorbornene. However, an atom(s) other than carbon atoms and hydrogen atoms may be contained at the end(s) of the polymer, or in a copolymerization component(s).

The medical material containing an olefinic polymer as a material of the substrate may be, for example, a separation membrane in the form of hollow fibers containing polypropylene or polymethylpentene, or a protective film containing polyethylene or polypropylene.

A substrate (a separation membrane or a film) incorporated in a commercially available medical device can be used as the substrate of the medical material, and the medical material can be produced, for example, by applying the coating agent to the substrate.

The present invention also provides a medical device incorporating the medical material.

Examples of the medical device incorporating the medical material include artificial kidney modules or plasma separators, blood purification devices represented by artificial lungs, blood circuits, blood bags, artificial joints, catheters, lead wires for pacemakers, etc., stents, stent-grafts, contact lenses and biosensors, that incorporate the separation membrane. Examples of the medical device using the medical material further include separation membrane modules for food and beverages that are used in contact with glycoproteins, and separation membrane modules used for the purification of biopharmaceuticals.

One embodiment of the medical device may be, for example, an artificial lung incorporating a separation membrane in the form of hollow fibers on the surface of which a layer has been formed by the coating agent, or an artificial heart-lung system incorporating the same.

Further, the present invention provides a method of coating the coating agent on the surface of the substrate. The method of carrying out the coating is as described above.

In addition, the present invention provides a method of producing a medical material with antithrombogenicity, the method including the step of coating the coating agent on the surface of the substrate. Specific embodiments of the step of the coating are as described above.

In the present invention, the antithrombogenicity of the medical material can be evaluated as follows. That is, the medical material is placed in a test container, immersed in human blood and shaken. After the medical material is retrieved and washed with saline, the proportion of the area of the thrombus-adhered region with respect to the entire surface of the retrieved medical material is calculated. Image processing is used for the calculation of the proportion of the area of the thrombus-adhered region, as will be described later.

The higher the antithrombogenicity of the medical material is, the lower the proportion of the area of the thrombus-adhered region is. When the antithrombogenicity is evaluated by the evaluation method described above, the proportion of the area of the thrombus-adhered region with respect to the surface of the medical material that have come into contact with human blood is preferably not more than 60%, more preferably not more than 50%, and still more preferably not more than 30%. The above-described proportion is most preferably 0%.

In cases where the shape of the medical material is flat, the proportion of the area of the thrombus-adhered region is evaluated for the surface of the medical material that have come into contact with human blood. In cases where the shape of the medical material is not flat, as well, the proportion of the area of the thrombus-adhered region with respect to the entire surface of the retrieved medical material is calculated, in the same manner. However, when it is difficult to perform an analysis by image processing directly due to reasons such as the shape of the medical material being greatly bent, image processing is carried out after peeling the adhered thrombi and placing them on a flat place, and then the proportion of the area of the thrombus-adhered region is calculated.

In cases where the medical material is a film or a separation membrane, the antithrombogenicity of the medical material can be evaluated by a platelet adhesion test, as well. That is, the medical material is fixed on a sample stage, immersed in human blood and shaken. After washing with saline, the surface of the medical material is observed by a scanning electron microscope, and the number of platelets adhered per unit area is counted. The above-described operation is carried out for 20 different visual fields, and the antithrombogenicity is evaluated by the mean value thereof.

The higher the antithrombogenicity of the medical material is, the smaller the number of platelets adhered is. When the antithrombogenicity is evaluated by the evaluation method described above, the number of platelets adhered is preferably not more than 25/4.3 × 10³ µm², more preferably not more than 20/4.3 × 10³ µm², and still more preferably not more than 10/4.3 × 10³ µm². The number of platelets adhered is most preferably 0/4.3 × 10³ µm².

In cases where the medical material is a hollow fiber membrane and when the antithrombogenicity of the inner surface of the hollow fiber membrane is evaluated, the evaluation is carried out by shaving the hollow fiber membrane to expose the inner surface thereof, and then immersing the membrane in human blood.

### EXAMPLES

The present invention is now described below by way of Examples. However, the present invention is not limited thereto.

### < Evaluation Methods >

### (1) NMR Measurement

The copolymer was added to 99.7% chloroform-D (with 0.05 V/V% TMS, manufactured by Wako Pure Chemical Industries, Ltd.) such that the concentration of the copolymer was 0.1% by mass, and dissolved. The resulting solution was placed in an NMR sample tube and subjected to an NMR measurement (a superconductive FT NMR, EX-270, manufactured by JEOL LTD.). The measurement was carried out at room temperature for a total of 32 times.

### (2) Antithrombogenicity Test

A polypropylene container (manufactured by AS ONE Corporation) having an inner diameter of 1.5 cm and a height of 1 cm was used as a test container. The medical material with a size of 1 cm square was placed in the test container, then 1 mL of human blood to which no anticoagulant had been added was added thereto, and the mixture was shaken at 100 rpm for 40 minutes. After the medical material is retrieved and washed with saline for 20 seconds, the proportion of the area of the thrombus-adhered region with respect to the entire surface of the retrieved medical material was calculated. The proportion of the area of the thrombus-adhered region was calculated as follows: the surface not in contact with the test container, of the surface of the retrieved medical material, was captured with a digital camera (PSD30, manufactured by Canon Inc.); the captured images were analyzed with analysis software, Image J (Image J bundled with 64-bit Java 1.8.0_112, manufactured by NIH); the area A of the entire medical material, and the area B which is the area of the region of the medical material to which thrombi had adhered were determined; and the proportion (B/A) of the area of the thrombus-adhered region was calculated by rounding off the digits after the decimal point.

### (3) Platelet Adhesion Test

A double-sided tape was pasted on a polystyrene disc plate with a diameter of 18 mm, and a hollow fiber membrane was fixed onto the tape. The pasted hollow fiber membrane was shaved into a half cylindrical shape with a single-edge blade, to expose the inner surface of the hollow fiber membrane. At this time, a hollow fiber membrane without fouling, cracks and folds was used, because, when there are any fouling, cracks and folds on the inner surface of the hollow fiber membrane, platelets may adhere to the portions therewith, possibly leading to a failure to perform accurate evaluation. The disc plate was attached to a Falcon (registered trademark) tube (No. 2051, diameter: 18 mm) which had been cut into a cylindrical shape such that the surface on which the hollow fiber membrane had been pasted faces the interior of the cylinder, and the gap was filled with a Parafilm. The interior of the cylindrical tube was washed with saline, and then filled with saline. Heparin was added to human blood to a concentration of 50 U/ml. After discarding the saline in the cylindrical tube, 1 mL of the blood was introduced into the cylindrical tube, and shaken at 37°C for 1 hour. Thereafter, the hollow fiber membrane was washed with 10 ml of saline, blood components were immobilized with 2.5% glutaraldehyde saline, followed by washing with 20 ml of distilled water. The washed hollow fiber membrane was dried at 20°C under a reduced pressure of 0.5 Torr for 10 hours. The dried hollow fiber membrane was pasted on the sample stage of a scanning electron microscope with a double-sided tape. Thereafter, a Pt-Pd thin film was formed on the surface of the hollow fiber membrane by sputtering, and the resulting membrane was used as a sample. The inner surface of the resulting sample of the hollow fiber membrane was observed by a field emission scanning electron microscope (S800, manufactured by Hitachi Ltd.) at a magnification of 1500, and the number of platelets adhered within the range of one visual field (4.3 × 10³ µm²) was counted. When not less than 50 platelets had adhered, it was determined that no effect of inhibiting the platelet adhesion was observed, and the number of platelets adhered was taken as 50. The number of platelets adhered was counted in 20 different visual fields, in the vicinity of the center of the hollow fiber membrane in the longitudinal direction thereof, and the mean value thereof was taken as the number of human platelets adhered (number/4.3 × 10³ µm²).

### (Example 1)

A quantity of 9.3 g of 3-(methyldimethoxysilyl)propyl methacrylate monomer (manufactured by Tokyo Chemical Industry Co., Ltd.), 8.0 g of vinyl propionate monomer (manufactured by Tokyo Chemical Industry Co., Ltd.), 8.1 g of vinylpyrrolidone monomer (manufactured by Wako Pure Chemical Industries, Ltd.), 41 g of amyl alcohol (TAA) (manufactured by Wako Pure Chemical Industries, Ltd.) as a polymerization solvent, and 0.10 g of azobisdimethylbutyronitrile (ADVN) (manufactured by Wako Pure Chemical Industries, Ltd.) as a polymerization initiator were mixed, and the mixture was stirred at 65°C for 6 hours under a nitrogen atmosphere. The reaction liquid was cooled to room temperature, and then introduced into hexane (manufactured by Wako Pure Chemical Industries, Ltd.). The white precipitates formed were collected, and dried under reduced pressure at 40°C for 12 hours, to obtain a 3-(methyldimethoxysilyl)propyl methacrylate/vinyl propionate/vinylpyrrolidone random copolymer (hereinafter, also referred to as "copolymer a"). Based on the results of ¹H-NMR measurement, the molar fraction of the 3-(methyldimethoxysilyl)propyl methacrylate monomer unit (hereinafter, also referred to as "unit A-1") with respect to the total amount of the copolymer was 36% by mole. Further, the molar fractions of the vinyl propionate monomer unit (corresponding to the unit B) and the vinylpyrrolidone monomer unit (corresponding to the unit C) with respect to the total amount of the copolymer were 24% by mole and 40% by mole, respectively. The total of the molar fractions of the unit A-1, the vinyl propionate monomer unit and the vinylpyrrolidone monomer unit with respect to the total amount of the copolymer was 100% by mole.

The antithrombogenicity test was carried out using the copolymer a as the coating agent. The copolymer a was dissolved in tetrahydrofuran (THF) (manufactured by Wako Pure Chemical Industries, Ltd.) such that the concentration of the copolymer a was 5% by mass, to prepare a coating solution. A PP film (manufactured by ALDRICH) cut out into a size of 1 cm square was immersed in the coating solution at 20°C for 2 minutes, the film was then retrieved and dried under vacuum at 20°C for 12 hours to produce a medical material.

The resulting medical material was subjected to the antithrombogenicity test, and as a result, the proportion of the area of the thrombus-adhered region was 2%, as shown in Table 1.

### (Example 2)

A quantity of 8.1 g of 3-[tris(trimethylsilyloxy)silyl]propyl methacrylate monomer (manufactured by Wako Pure Chemical Industries, Ltd.), 1.9 g of 2-hydroxyethyl methacrylate monomer (manufactured by Wako Pure Chemical Industries, Ltd.), 25 g of THF and 0.31 g of ADVN were mixed, and the mixture was stirred at 60°C for 4 hours under a nitrogen atmosphere, to obtain a solution of a 3-[tris(trimethylsilyloxy)silyl]propyl methacrylate/2-hydroxyethyl methacrylate random copolymer. Separately, 9.0 g of vinyl propionate monomer, 9.0 g of vinylpyrrolidone monomer, 0.18 g of acrylic acid monomer, 50 g of THF and 0.10 g of ADVN were mixed, and the mixture was stirred at 60°C for 2.5 hours under a nitrogen atmosphere, to obtain a solution of a vinyl propionate/vinylpyrrolidone/acrylic acid random copolymer. The two resulting solutions were mixed, and to the mixture were added 0.85 g of N,N'-dicyclohexylcarbodiimide (DCC) (manufactured by Wako Pure Chemical Industries, Ltd.), 0.25 g of 4,4-dimethylaminopyridinium p-toluenesulfonate (DPTS) (manufactured by FLUOROCHEM) and 0.10 g of 4,4-dimethylaminopyridine (DMAP) (manufactured by Wako Pure Chemical Industries, Ltd.). The resulting mixture was stirred at 60°C for 4 hours to induce the condensation of the hydroxyl group of the 2-hydroxyethyl methacrylate and the carboxy group of the acrylic acid. The reaction liquid was cooled to room temperature, and then introduced into water. The white precipitates formed were collected and dried under reduced pressure at 30°C for 24 hours, to obtain a 3-[tris(trimethylsilyloxy)silyl]propyl methacrylate/2-hydroxyethyl methacrylate-vinyl propionate/vinylpyrrolidone/acrylic acid graft copolymer (hereinafter, also referred to as "copolymer b"). Based on the results of ¹H-NMR measurement, the molar fraction of the 3-[tris(trimethylsilyloxy)silyl]propyl methacrylate monomer unit (hereinafter, also referred to as "unit A-2") with respect to the total amount of the copolymer was 37% by mole. Further, the molar fraction of the vinyl propionate monomer unit (corresponding to the unit B) with respect to the total amount of the copolymer was 19% by mole; and the total of the molar fractions of the 2-hydroxyethyl methacrylate monomer unit, the vinylpyrrolidone monomer unit and the acrylic acid monomer unit (corresponding to the units C) with respect to the total amount of the copolymer was 44% by mole. The total of the molar fraction of the unit A-2, the molar fraction of the vinyl propionate monomer unit, as well as the molar fractions of the 2-hydroxyethyl methacrylate monomer unit, the vinylpyrrolidone monomer unit and the acrylic acid monomer unit with respect to the total amount of the copolymer was 100% by mole.

A medical material was produced in the same manner as in Example 1, except for using the copolymer b as the coating agent.

The resulting medical material was subjected to the antithrombogenicity test, and as a result, the proportion of the area of the thrombus-adhered region was 45%, as shown in Table 1.

### (Example 3)

A quantity of 1.8 g of vinyl propionate monomer, 1.8 g of vinylpyrrolidone monomer, 0.07 g of acrylic acid monomer, 15 g of dimethyl sulfoxide (DMSO) (manufactured by Wako Pure Chemical Industries, Ltd.) and 0.05 g of ADVN were mixed, and the resulting mixture was stirred at 65°C for 2 hours under a nitrogen atmosphere, to obtain a solution of a vinyl propionate/vinylpyrrolidone/acrylic acid random copolymer. To the resulting solution, 1.3 g of a propylmethacryl-heptaisobutyl-polyhedral oligomeric silsesquioxane (PSS)/2-hydroxymethyl methacrylate copolymer (manufactured by Sigma-Aldrich Inc.), 45 g of DMSO, 0.23 g of DCC, 0.09 g of DPTS and 0.05 g of DMAP were introduced and mixed. The resulting mixture was stirred at 60°C for 4 hours to induce the condensation of the hydroxyl group of the 2-hydroxymethyl methacrylate monomer unit and the carboxy group of the acrylic acid. The reaction liquid was cooled to room temperature, and then introduced into water. The white precipitates formed were collected and dried under reduced pressure at 30°C for 24 hours, to obtain a propylmethacryl-heptaisobutyl-PSS/2-hydroxymethyl methacrylate-vinyl propionate/vinylpyrrolidone/acrylic acid graft copolymer (hereinafter, also referred to as "copolymer c"). Based on the results of ¹H-NMR measurement, the molar fraction of the propylmethacryl-heptaisobutyl-PSS monomer unit (hereinafter, also referred to as "unit A-3") with respect to the total amount of the copolymer was 53% by mole.

A medical material was produced in the same manner as in Example 1, except for using the copolymer c as the coating agent.

The resulting medical material was subjected to the antithrombogenicity test, and as a result, the proportion of the area of the thrombus-adhered region was 34%, as shown in Table 1.

### (Example 4)

A quantity of 7.1 g of 3-[3-(9-butyl-1,1,3,3,5.5,7,7,9,9-decamethyl-1-pentasiloxanyl)propoxyl]-2-hydroxyl propyl methacrylate monomer (manufactured by Toray Industries, Inc.), 1.9 g of 2-hydroxyethyl methacrylate monomer (manufactured by Wako Pure Chemical Industries, Ltd.), 19 g of THF and 0.09 g of ADVN were mixed, and the mixture was stirred at 60°C for 2 hours under a nitrogen atmosphere, to obtain a solution of a 3-[3-(9-butyl-1,1,3,3,5,5,7,7,9,9-decamethyl-1-pentasiloxanyl)propoxyl]-2-hydroxyl propyl methacrylate/2-hydroxyethyl methacrylate random copolymer. Separately, 9.0 g of vinyl propionate monomer, 9.0 g of vinylpyrrolidone monomer, 0.18 g of acrylic acid monomer, 50 g of THF and 0.10 g of ADVN were mixed, and the mixture was stirred at 60°C for 2.5 hours under a nitrogen atmosphere, to obtain a solution of a vinyl propionate/vinylpyrrolidone/acrylic acid random copolymer. The two resulting solutions were mixed, and to the mixture were added 0.85 g of DCC, 0.25 g of DPTS and 0.10 g of DMAP. The resulting mixture was stirred at 60°C for 4 hours to induce the condensation of the hydroxyl group of the 2-hydroxyethyl methacrylate and the carboxy group of the acrylic acid. The reaction liquid was cooled to room temperature, and then introduced into water. The white precipitates formed were collected and dried under reduced pressure at 30°C for 24 hours, to obtain a
3-[3-(9-butyl-1,1,3,3,5,5,7,7,9,9-decamethyl-1-pentasiloxanyl)propoxyl]-2-hydroxyl propyl methacrylate/2-hydroxyethyl methacrylate-vinyl propionate/vinylpyrrolidone/acrylic acid graft copolymer (hereinafter, also referred to as "copolymer d"). Based on the results of ¹H-NMR measurement, the molar fraction of the 3-[3-(9-butyl-1,1,3,3,5,5,7,7,9,9-decamethyl-1-pentasiloxanyl)propoxyl]-2-hydroxyl propyl methacrylate monomer unit (hereinafter, also referred to as "unit A-4") with respect to the total amount of the copolymer was 13% by mole.

A medical material was produced in the same manner as in Example 1, except for using the copolymer d as the coating agent.

The resulting medical material was subjected to the antithrombogenicity test, and as a result, the proportion of the area of the thrombus-adhered region was 37%, as shown in Table 1.

### (Example 5)

A quantity of 1.0 g of 3-[tris(trimethylsilyloxy)silyl]propyl methacrylate monomer, 1.0 g of vinyl hexanoate monomer, 5.0 g of vinylacetamide monomer, 25 g of TAA and 0.05 g of ADVN were mixed, and the mixture was stirred at 65°C for 4 hours under a nitrogen atmosphere. The reaction liquid was cooled to room temperature, and then introduced into water. The white precipitates formed were collected and dried under reduced pressure at 30°C for 24 hours, to obtain a 3-[tris(trimethylsilyloxy)silyl]propyl methacrylate/vinyl hexanoate/vinylacetamide random copolymer (hereinafter, also referred to as "copolymer e"). Based on the results of ¹H-NMR measurement, the molar fraction of the unit A-2 with respect to the total amount of the copolymer was 9% by mole.

A medical material was produced in the same manner as in Example 1, except for using the copolymer e as the coating agent.

The resulting medical material was subjected to the antithrombogenicity test, and as a result, the proportion of the area of the thrombus-adhered region was 16%, as shown in Table 1.

### (Example 6)

A quantity of 1.0 g of 3-[tris(trimethylsilyloxy)silyl]propyl methacrylate monomer, 3.0 g of vinyl hexanoate monomer, 5.0 g of vinylpyrrolidone monomer, 25 g of TAA and 0.08 g of ADVN were mixed, and the mixture was stirred at 65°C for 4 hours under a nitrogen atmosphere. The reaction liquid was cooled to room temperature, and then introduced into water. The white precipitates formed were collected and dried under reduced pressure at 30°C for 24 hours, to obtain a 3-[tris(trimethylsilyloxy)silyl]propyl methacrylate/vinyl hexanoate/vinylpyrrolidone random copolymer (hereinafter, also referred to as "copolymer f"). Based on the results of ¹H-NMR measurement, the molar fraction of the unit A-2 with respect to the total amount of the copolymer was 15% by mole. Further, the molar fractions of the vinyl hexanoate monomer unit (corresponding to the unit B) and the vinylpyrrolidone monomer unit (corresponding to the unit C) with respect to the total amount of the copolymer were 24% by mole and 61% by mole, respectively. The total of the molar fractions of the unit A-2, the vinyl hexanoate monomer unit and the vinylpyrrolidone monomer unit with respect to the total amount of the copolymer unit was 100% by mole.

A medical material was produced in the same manner as in Example 1, except for using the copolymer f as the coating agent.

The resulting medical material was subjected to the antithrombogenicity test, and as a result, the proportion of the area of the thrombus-adhered region was 31%, as shown in Table 1.

### (Example 7)

A quantity of 4.0 g of 3-[tris(trimethylsilyloxy)silyl]propyl methacrylate monomer, 5.0 g of vinyl propionate monomer, 5.5 g of vinylpyrrolidone monomer, 25 g of TAA and 0.05 g of ADVN were mixed, and the mixture was stirred at 65°C for 4 hours under a nitrogen atmosphere. The reaction liquid was cooled to room temperature, and then introduced into water. The white precipitates formed were collected and dried under reduced pressure at 30°C for 24 hours, to obtain a 3-[tris(trimethylsilyloxy)silyl]propyl methacrylate/vinyl propionate/vinylpyrrolidone random copolymer (hereinafter, also referred to as "copolymer g"). Based on the results of ¹H-NMR measurement, the molar fraction of the unit A-2 with respect to the total amount of the copolymer was 27% by mole. Further, the molar fractions of the vinyl propionate monomer unit (corresponding to the unit B) and the vinylpyrrolidone monomer unit (corresponding to the unit C) with respect to the total amount of the copolymer were 22% by mole and 51% by mole, respectively. The total of the molar fractions of the unit A-2, the vinyl propionate monomer unit and the vinylpyrrolidone monomer unit with respect to the total amount of the copolymer was 100% by mole.

A medical material was produced in the same manner as in Example 1, except for using the copolymer g as the coating agent.

The resulting medical material was subjected to the antithrombogenicity test, and as a result, the proportion of the area of the thrombus-adhered region was 2%, as shown in Table 1.

### (Example 8)

A solution of a vinyl propionate/vinylpyrrolidone/acrylic acid random copolymer was obtained in the same manner as in Example 3. A quantity of 5.0 g of the resulting solution was collected, mixed with 0.30 g of a poly[dimethylsiloxane/[3-(2-(2-hydroxyethoxy)ethoxy)propyl]methylsiloxane] copolymer (manufactured by ALDRICH), 0.10 g of DCC, 0.02 g of DPTS and 0.01 g of DMAP, and the mixture was stirred at 30°C for 4 hours. The reaction liquid was introduced into water, and the white precipitates formed were collected and dried under reduced pressure at 30°C for 24 hours, to obtain a dimethylsiloxane-vinyl propionate/vinylpyrrolidone/acrylic acid graft copolymer (hereinafter, also referred to as "copolymer h"). Based on the results of ¹H-NMR measurement, the molar fraction of the dimethylsiloxane monomer unit (hereinafter, also referred to as "unit A-5") with respect to the total amount of the copolymer was 35% by mole.

A medical material was produced in the same manner as in Example 1, except for using the copolymer h as the coating agent.

The resulting medical material was subjected to the antithrombogenicity test, and as a result, the proportion of the area of the thrombus-adhered region was 48%, as shown in Table 1.

### (Comparative Example 1)

A PP film (manufactured by ALDRICH) cut out into a size of 1 cm square was used as it is, as the medical material, and subjected to the antithrombogenicity test. As a result, the proportion of the area of the thrombus-adhered region was 100%, as shown in Table 2.

### (Comparative Example 2)

A quantity of 17.5 g of vinyl propionate monomer, 19.5 g of vinylpyrrolidone monomer, 56 g of THF and 0.175 g of ADVN were mixed, and the mixture as stirred at 70°C for 5 hours under a nitrogen atmosphere. The reaction liquid was cooled to room temperature to terminate the reaction, and then introduced into hexane. The white precipitates formed were collected and dried under reduced pressure, to obtain a vinyl propionate/vinylpyrrolidone random copolymer (hereinafter, also referred to as "copolymer i").

A medical material was produced in the same manner as in Example 1, except that the copolymer i was used as the coating agent instead of the copolymer a, and that water was used instead of THF.

The resulting medical material was subjected to the antithrombogenicity test, and as a result, the proportion of the area of the thrombus-adhered region was 73%, as shown in Table 2.

### (Comparative Example 3)

The solution of the 3-[tris(trimethylsilyloxy)silyl]propyl methacrylate/2-hydroxyethyl methacrylate random copolymer prepared in Example 2 was introduced into water. The white precipitates formed were collected and dried under reduced pressure at 30°C for 24 hours, to obtain a poly(3-[tris(trimethylsilyloxy)silyl]propyl methacrylate/2-hydroxyethyl methacrylate) random copolymer (hereinafter, also referred to as "copolymer j").

A medical material was produced in the same manner as in Example 1, except that the copolymer j was used as the coating agent instead of the copolymer a, and that the copolymer concentration in the solution was changed to 1% by mass.

The resulting medical material was subjected to the antithrombogenicity test, and as a result, the proportion of the area of the thrombus-adhered region was 100%, as shown in Table 2.

### (Comparative Example 4)

The solution of the 3-[3-(9-butyl-1,1,3,3,5,5,7,7,9,9-decamethyl-1-pentasiloxanyl)propoxyl]-2-hydroxyl propyl methacrylate/2-hydroxyethyl methacrylate random copolymer prepared in Example 4 was introduced into water. The white precipitates formed were collected and dried under reduced pressure at 30°C for 24 hours, to obtain a 3-[3-(9-butyl-1,1,3,3,5,5,7,7,9.9-decamethyl-1-pentasiloxanyl)propoxyl]-2-hydroxyl propyl methacrylate/2-hydroxyethyl methacrylate random copolymer (hereinafter, also referred to as "copolymer k").

A medical material was produced in the same manner as in Example 1, except that the copolymer k was used as the coating agent instead of the copolymer a, and that the copolymer concentration in the solution was changed to 1% by mass.

The resulting medical material was subjected to the antithrombogenicity test, and as a result, the proportion of the area of the thrombus-adhered region was 100%, as shown in Table 2.

### (Example 9)

A medical material was produced using the copolymer a synthesized in Example 1 as the coating agent, and using a polycarbonate (PC) plate (manufactured by ALDRICH) instead of the PP film.

The resulting medical material was subjected to the antithrombogenicity test, and as a result, the proportion of the area of the thrombus-adhered region was 13%, as shown in Table 1.

### (Comparative Example 5)

A PC plate cut out into a size of 1 cm square was used as it is, as the medical material, and subjected to the antithrombogenicity test. As a result, the proportion of the area of the thrombus-adhered region was 100%, as shown in Table 2.

### (Example 10)

A medical material was produced using the copolymer a synthesized in Example 1 as the coating agent, and using a polymethylpentene (PMP) plate (manufactured by Kartell) instead of the PP film.

The resulting medical material was subjected to the antithrombogenicity test, and as a result, the proportion of the area of the thrombus-adhered region was 1 %, as shown in Table 1.

### (Example 11)

A medical material was produced using the copolymer g synthesized in Example 7 as the coating agent, and using a PMP plate instead of the PP film.

The resulting medical material was subjected to the antithrombogenicity test, and as a result, the proportion of the area of the thrombus-adhered region was 1%, as shown in Table 1.

### (Example 12)

A medical material was produced using the copolymer d synthesized in Example 4 as the coating agent, and using a PMP plate instead of the PP film.

The resulting medical material was subjected to the antithrombogenicity test, and as a result, the proportion of the area of the thrombus-adhered region was 37%, as shown in Table 1.

### (Comparative Example 6)

A PMP plate cut out into a size of 1 cm square was used as it is, as the medical material, and subjected to the antithrombogenicity test. As a result, the proportion of the area of the thrombus-adhered region was 100%, as shown in Table 2.

### (Example 13)

A medical material was produced using the copolymer a synthesized in Example 1 as the coating agent, and using a polyvinyl chloride (PVC) plate which had been prepared by cutting out a chamber of a blood circuit (manufactured by Ilanaco medical Co., Ltd.) into a size of 1 cm square, instead of the PP film.

The resulting medical material was subjected to the antithrombogenicity test, and as a result, the proportion of the area of the thrombus-adhered region was 26%, as shown in Table 1.

### (Comparative Example 7)

A PVC plate cut out into a size of 1 cm square was used as it is, as the medical material, and subjected to the antithrombogenicity test. As a result, the proportion of the area of the thrombus-adhered region was 87%, as shown in Table 2.

### (Example 14)

A medical material was produced using the copolymer a synthesized in Example 1 as the coating agent, and using a silicone (Si) plate (manufactured by AS ONE Corporation) instead of the PP film.

The resulting medical material was subjected to the antithrombogenicity test, and as a result, the proportion of the area of the thrombus-adhered region was 18%, as shown in Table 1.

### (Comparative Example 8)

A Si plate cut out into a size of 1 cm square was used as it is, as the medical material, and subjected to the antithrombogenicity test. As a result, the proportion of the area of the thrombus-adhered region was 100%, as shown in Table 2.

### (Example 15)

A quantity of 1.0 g of 3-[3-(9-butyl-1,1,3,3,5,5,7,7,9,9-decamethyl-1-pentasiloxanyl)propoxyl]-2-hydroxyl propyl methacrylate monomer, 1.0 g of vinyl acetate monomer (manufactured by Wako Pure Chemical Industries, Ltd.), 5.0 g of vinylpyrrolidone monomer, 25 g of TAA and 0.05 g of ADVN were mixed, and the mixture was stirred at 65°C for 4 hours under a nitrogen atmosphere. The reaction liquid was cooled to room temperature, and then introduced into water. The white precipitates formed were collected and dried under reduced pressure at 30°C for 24 hours, to obtain a 3-[3-(9-butyl-1,1,3,3,5,5,7,7,9,9-decamethyl-1-pentasiloxanyl)propoxyl]-2-hydroxyl propyl methacrylate/vinyl acetate/vinylpyrrolidone random copolymer (hereinafter, also referred to as "copolymer 1"). Based on the results of ¹H-NMR measurement, the molar fraction of the unit A-4 with respect to the total amount of the copolymer was 25% by mole. Further, the molar fractions of the vinyl acetate monomer unit (corresponding to the unit B) and the vinylpyrrolidone monomer unit (corresponding to the unit C) with respect to the total amount of the copolymer were 30% by mole and 45% by mole, respectively. The total of the molar fractions of the unit A-4, the vinyl acetate monomer unit and the vinylpyrrolidone monomer unit with respect to the total amount of the copolymer was 100% by mole. The solubility of the copolymer 1 in 100 g of water at 20°C was less than 5 g, indicating that the copolymer was poorly water-soluble.

A medical material was produced in the same manner as in Example 1, except for using the copolymer 1 as the coating agent.

The resulting medical material was subjected to the antithrombogenicity test, and as a result, the proportion of the area of the thrombus-adhered region was 22%, as shown in Table 1.

### (Example 16)

A quantity of 7.1 g of 3-(methyldimethoxysilyl)propyl methacrylate monomer, 1.9 g of 2-hydroxyethyl methacrylate monomer, 19 g of THF and 0.09 g of ADVN were mixed, and the mixture was stirred at 60°C for 2 hours under a nitrogen atmosphere, to obtain a solution of a 3-(methyldimethoxysilyl)propyl methacrylate/2-hydroxyethyl methacrylate random copolymer. Separately, 9.0 g of vinyl butyrate monomer (manufactured by Tokyo Chemical Industry Co., Ltd.), 9.0 g of vinylpyrrolidone monomer, 0.18 g of acrylic acid monomer, 50 g of THF and 0.10 g of ADVN were mixed, and the mixture was stirred at 60°C for 2.5 hours under a nitrogen atmosphere, to obtain a solution of a vinyl butyrate/vinylpyrrolidone/acrylic acid random copolymer. The two resulting solutions were mixed, and to the mixture were added 0.85 g of DCC, 0.25 g of DPTS and 0.10 g of DMAP. The resulting mixture was stirred at 60°C for 4 hours to induce the condensation of the hydroxyl group of the 2-hydroxyethyl methacrylate and the carboxy group of the acrylic acid. The reaction liquid was cooled to room temperature, and then introduced into water. The white precipitates formed were collected and dried under reduced pressure at 30°C for 24 hours, to obtain a 3-(methyldimethoxysilyl)propyl methacrylate/2-hydroxyethyl methacrylate-vinyl butyrate/vinylpyrrolidone/acrylic acid graft copolymer (hereinafter, also referred to as "copolymer m"). Based on the results of ¹H-NMR measurement, the molar fraction of the unit A-1 with respect to the total amount of the copolymer was 16% by mole.

A medical material was produced in the same manner as in Example 1, except for using the copolymer m as the coating agent.

The resulting medical material was subjected to the antithrombogenicity test, and as a result, the proportion of the area of the thrombus-adhered region was 44%, as shown in Table 1.

### (Example 17)

A quantity of 1.0 g of 3-[tris(trimethylsilyloxy)silyl]propyl methacrylate monomer, 1.0 g of vinyl pivalate (manufactured by Tokyo Chemical Industry Co., Ltd.), 5.0 g of acrylamide monomer (manufactured by Wako Pure Chemical Industries, Ltd.), 25 g of TAA and 0.05 g of ADVN were mixed, and the mixture was stirred at 65°C for 4 hours under a nitrogen atmosphere. The reaction liquid was cooled to room temperature, and then introduced into water. The white precipitates formed were collected and dried under reduced pressure at 30°C for 24 hours, to obtain a 3-[tris(trimethylsilyloxy)silyl]propyl methacrylate/vinyl pivalate/acrylamide random copolymer (hereinafter, also referred to as "copolymer n"). Based on the results of ¹H-NMR measurement, the molar fraction of the unit A-2 with respect to the total amount of the copolymer was 10% by mole.

A medical material was produced in the same manner as in Example 1, except for using the copolymer n as the coating agent.

The resulting medical material was subjected to the antithrombogenicity test, and as a result, the proportion of the area of the thrombus-adhered region was 14%, as shown in Table 1.

**[Table 1]**

| | Copolymer | Unit A | Unit B | Unit C | Monomer Arrangement | Substrate | Area of Thrombus-adhered Region (%) |
|---|---|---|---|---|---|---|---|
| Example 1 | Copolymer a | Unit A-1 | Vinyl propionate | Vinylpyrrolidone | Random | PP | 2 |
| Example 2 | Copolymer b | Unit A-2 | Vinyl propionate | Vinylpyrrolidone, 2-hydroxyethyl Methacrylate Acrylic acid | Graft | PP | 45 |
| Example 3 | Copolymer c | Unit A-3 | Vinyl propionate | Vinylpyrrolidone, 2-hydroxyethyl Methacrylate Acrylic acid | Graft | PP | 34 |
| Example 4 | Copolymer d | Unit A-4 | Vinyl propionate | Vinylpyrrolidone, 2-hydroxyethyl Methacrylate Acrylic acid | Graft | PP | 37 |
| Example 5 | Copolymer e | Unit A-2 | Vinyl hexanoate | Vinylacetamide | Random | PP | 16 |
| Example 6 | Copolymer f | Unit A-2 | Vinyl hexanoate | Vinylpyrrolidone | Random | PP | 31 |
| Example 7 | Copolymer g | Unit A-2 | Vinyl propionate | Vinylpyrrolidone | Random | PP | 2 |
| Example 8 | Copolymer h | Unit A-5 | Vinyl propionate | Vinylpyrrolidone, Acrylic acid | Graft | PP | 48 |
| Example 9 | Copolymer a | Unit A-1 | Vinyl propionate | Vinylpyrrolidone | Random | PC | 13 |
| Example 10 | Copolymer a | Unit A-1 | Vinyl propionate | Vinylpyrrolidone | Random | PMP | 1 |
| Example 11 | Copolymer g | Unit A-2 | Vinyl propionate | Vinylpyrrolidone | Random | PMP | 1 |
| Example 12 | Copolymer d | Unit A-4 | Vinyl propionate | Vinylpyrrolidone, 2-hydroxyethyl Methacrylate Acrylic acid | Graft | PMP | 37 |
| Example 13 | Copolymer a | Unit A-1 | Vinyl propionate | Vinylpyrrolidone | Random | PVC | 26 |
| Example 14 | Copolymer a | Unit A-1 | Vinyl propionate | Vinylpyrrolidone | Random | Si | 18 |
| Example 15 | Copolymer 1 | Unit A-4 | Vinyl acetate | Vinylpyrrolidone | Random | PP | 22 |
| Example 16 | Copolymer m | Unit A-1 | Vinyl acetate | Vinylpyrrolidone, 2-hydroxyethyl Methacrylate Acrylic acid | Graft | PP | 44 |
| Example 17 | Copolymer n | Unit A-2 | Vinyl pivalate | Acrylamide | Random | PP | 14 |

**[Table 2]**

| | Copolymer | Unit A | Unit B | Unit C | Monomer Arrangement | Substrate | A rea of Thrombus-adhered Region (%) |
|---|---|---|---|---|---|---|---|
| Comparative Example 1 | - | - | - | - | - | PP | 100 |
| Comparative Example 2 | Copolymer i | - | Vinyl propionate | Vinylpyrrolidone | Random | PP | 73 |
| Comparative Example 3 | Copolymer j | Unit A-2 | - | Methacrylic acid 2-hydroxyethyl | Random | PP | 100 |
| Comparative Example 4 | Copolymer k | Unit A-4 | - | Methacrylic acid 2-hydroxyethyl | Random | PP | 100 |
| Comparative Example 5 | - | - | - | - | - | PC | 100 |
| Comparative Example 6 | - | - | - | - | - | PMP | 100 |
| Comparative Example 7 | - | - | - | - | - | PVC | 87 |
| Comparative Example 8 | - | - | - | - | - | Si | 100 |

In Table 1 and Table 2, the unit A-1 represents 3-(methyldimethoxysilyl)propyl methacrylate monomer unit, the unit A-2 represents 3-[tris(trimethylsilyloxy)silyl]propyl methacrylate monomer unit, the unit A-3 represents propylmethacryl-heptaisobutyl-PSS monomer unit, the unit A-4 represents 3-[3-(9-butyl-1,1,3,3,5,5,7,7,9,9-decamethyl-1-pentasiloxanyl)propoxyl]-2-hydroxyl propyl methacrylate monomer unit, and the unit A-5 represents dimethylsiloxane monomer unit. Further, PP represents polypropylene, PC represents polycarbonate, PMP represents polymethylpentene, PVC represents polyvinyl chloride, and Si represents silicone.

### (Example 18)

The copolymer g was dissolved in THF such that the concentration of the copolymer g was 1% by mass, to prepare a coating solution. A PP hollow fiber membrane (manufactured by 3M Ltd.) cut out in a length of 2 cm was immersed in the coating solution at 20°C for 2 minutes, retrieved, and dried under vacuum at 20°C for 12 hours to produce a medical material. At the time of immersion, care was taken to allow the coating solution to penetrate into the inner side of the hollow fiber membrane.

The resulting medical material was subjected to the platelet adhesion test, and as a result, the number of platelets adhered was one, as shown in Table 3.

### (Example 19)

A medical material was produced in the same manner as in Example 18, except that the copolymer b was used instead of the copolymer g.

The resulting medical material was subjected to the platelet adhesion test, and as a result, the number of platelets adhered was 19, as shown in Table 3.

### (Comparative Example 9)

A PP hollow fiber membrane was used as it is, as the medical material, and subjected to the platelet adhesion test. As a result, the number of platelets adhered was 46, as shown in Table 3.

**[Table 3]**

| | Copolymer | Unit A | Unit B | Unit C | Monomer Arrangement | Substrate | Number of Platelets Adhered (number/visual field) |
|---|---|---|---|---|---|---|---|
| Example 18 | Copolymer g | Unit A-2 | Vinyl propionate | Vinylpyrrolidone | Random | PP hollow fiber membrane | 1 |
| Example 19 | Copolymer b | Unit A-2 | Vinyl propionate | Vinylpyrrolidone, 2-hydroxyethyl Methacrylate Acrylic acid | Graft | PP hollow fiber membrane | 19 |
| Comparative Example 9 | - | - | - | - | - | PP hollow fiber membrane | 46 |

In Table 3, the unit A-2 represents 3-[tris(trimethylsilyloxy)silyl]propyl mcthacrylate monomer unit, and PP represents polypropylene.

The above results revealed that the use of the coating agent of the present invention enables to easily give antifouling properties, particularly, antithrombogenicity, to the medical material, and further, to give a particularly high antithrombogenicity thereto when the respective units constituting the copolymer contained in the coating agent are arranged randomly.

### INDUSTRIAL APPLICABILITY

The coating agent of the present invention is capable of giving a high antithrombogenicity to a wide range of medical materials and the like.

## Claims

1. A coating agent comprising a copolymer, the copolymer comprising:
a monomer unit (unit A) containing a Si-O bond;
a vinyl carboxylate monomer unit (unit B); and
a monomer unit (unit C) containing a hydrophilic group.

2. The coating agent according to claim 1, wherein the unit A is a monomer unit that contains a group selected from the group consisting of alkylalkoxysilyl groups, tris(trialkylsilyloxy)silyl groups and dialkylsiloxane groups.

3. The coating agent according to claim 1 or 2, wherein the unit B is a unit selected from the group consisting of vinyl acetate monomer unit, vinyl propionate monomer unit, vinyl butyrate monomer unit, vinyl pentanoate monomer unit, vinyl pivalate monomer unit and vinyl hexanoate monomer unit.

4. The coating agent according to any one of claims 1 to 3, wherein the hydrophilic group is an amide group.

5. The coating agent according to any one of claims 1 to 4, wherein the units A, B and C are arranged randomly in the copolymer.

6. A medical material comprising:
a substrate; and
a layer formed on the surface of the substrate by the coating agent according to any one of claims 1 to 5.

7. The medical material according to claim 6, wherein the substrate is made of a material containing an olefinic polymer.

8. A copolymer comprising:
a monomer unit (unit A) containing a Si-O bond;
a vinyl carboxylate monomer unit (unit B); and
a monomer unit (unit C) containing a hydrophilic group;
wherein the unit A is a monomer unit that contains a group selected from the group consisting of alkylalkoxysilyl groups, tris(trialkylsilyloxy)silyl groups and dialkylsiloxane groups.

9. The copolymer according to claim 8,
wherein the unit B is a unit selected from the group consisting of vinyl acetate monomer unit, vinyl propionate monomer unit, vinyl butyrate monomer unit, vinyl pentanoate monomer unit, vinyl pivalate monomer unit and vinyl hexanoate monomer unit; and
wherein the unit C is vinylpyrrolidone monomer unit, vinylacetamide monomer unit or acrylamide monomer unit.

10. The copolymer according to claim 8 or 9, wherein the units A, B and C are arranged randomly in the copolymer.
